# EUROPEAN PATENT SPECIFICATION

(11) **EP 4 151 099 B1**
(45) Date of publication and mention of the grant of the patent: **17.12.2025**
(21) Application number: 20935518.9
(22) Date of filing: 12.05.2020
(51) Int. Cl.: A24F 40/40, A61M 11/04, A61M 15/06, A24F 40/10, A24F 40/44, A24F 40/46

(54) **ATOMIZATION DEVICE**
ZERSTÄUBUNGSVORRICHTUNG
DISPOSITIF D'ATOMISATION

(43) Date of publication of application: 22.03.2023
(60) Divisional application: 25202576.2
(73) Proprietor: Shenzhen Smoore Technology Limited, Shenzhen, Guangdong 518102 (CN)
(72) Inventor: LEI, Guilin, Shenzhen, Guangdong 518102 (CN); JIANG, Ru, Shenzhen, Guangdong 518102 (CN)
(74) Representative: Herrero & Asociados, S.L.
(86) International application number: PCT/CN2020/089824
(87) International publication number: WO 2021/226834

(56) References cited:
- WO-A1-2018/161254
- WO-A1-2019/234195
- WO-A1-2020/064921
- CN-A- 109 310 152
- CN-A- 110 352 017
- CN-A- 110 613 171
- CN-A- 110 613 171
- CN-A- 110 613 172
- US-A1- 2015 027 470

## Description

### TECHNICAL FIELD

The present disclosure relates to the technical field of atomization devices, and in particular, to an atomization device.

### BACKGROUND

Currently, a cavity wall of an atomization cavity in an atomization device such as an e-cigarette is made of a plastic material. When the temperature in the atomization cavity is excessively high, stability problems such as deformation, and even scorching of the cavity wall of the atomization cavity are usually caused. Moreover, when the atomization device heats and atomizes an aerosol substrate such as e-liquid, an oil frying phenomenon is prone to occur due to uneven distribution of the aerosol substrate and high local temperature. The non-atomized aerosol substrate splashes to the cavity wall of the atomization cavity, or the atomized aerosol substrate condenses on the cavity wall of the atomization cavity. The aerosol substrate attached to the cavity wall of the atomization cavity may reach the bottom of the atomization device along the cavity wall, causing a liquid leakage problem.

PCT application WO2019/234195A1 discloses an aerosol-generating device (100). The aerosol-generating device (100) comprises: a storage container (10) containing a liquid aerosol-forming substrate; a heating element (14) spaced from the storage container (10) for vaporising the aerosol-forming substrate to form an aerosol; a transfer element (16) located between the storage container (10) and the heating element (14), adapted to receive and retain aerosol-forming substrate from the storage container (100, and capable of being coupled with the heating element (14) to supply the received aerosol-forming substrate to the heating element (14). At least one of the transfer element (16) and the heating element (14) is movable between a loading position and a heating position. With the movable element(s) in the loading position, the transfer element (16) is in fluid communication with the outlet of the storage container (10) or with the outlet of the storage container (10) and with the heating element (14). With the movable element(s) in the heating position, the transfer element (16) is in fluid communication with the heating element (14) and the heating element (14) is activated to vaporise aerosol-forming substrate retained in the transfer element (16).

### SUMMARY

In view of this, a technical problem to be mainly resolved by the present disclosure is to provide an atomization device, which may improve the structural stability of the atomization device and improve the anti-leakage effect of the atomization device.

To resolve the foregoing technical problem, a technical solution adopted by the present invention is to provide an atomization device as defined in the appended claims. The atomization device includes an air inlet, an air outlet, and an atomization cavity. The atomization cavity is in communication with the air inlet and the air outlet respectively, and an atomization assembly is arranged in the atomization cavity. A first capillary liquid absorbing structure is defined on the part of the inner wall of the atomization cavity close to the atomization assembly. After the first capillary liquid absorbing structure absorbs an aerosol substrate, the temperature of the cavity wall of the atomization cavity at the position where the first capillary liquid absorbing structure is located is lowered with endothermic atomization of the aerosol substrate.

In some embodiments of the present disclosure, the atomization device further includes a first carrier and a second carrier. The atomization cavity is formed by docking the first carrier and the second carrier. The air inlet is provided on the first carrier. The atomization assembly is arranged on the second carrier, and the first capillary liquid absorbing structure is defined on the inner wall of the second carrier.

In some embodiments of the present disclosure, the first capillary liquid absorbing structure is defined on the side wall of the second carrier.

In some embodiments of the present disclosure, when the atomization assembly generates heat, the temperature of the cavity wall of the atomization cavity is less than or equal to 150°C.

In some embodiments of the present disclosure, the distance between the surface of the atomization assembly and the inner wall of the atomization cavity ranges from 0.5 mm to 1.8 mm.

In some embodiments of the present disclosure, the distance between the surface of the atomization assembly and the inner wall of the atomization cavity ranges from 1 mm to 1.5 mm.

In some embodiments of the present disclosure, the atomization device further includes an air outlet channel. The air outlet channel is in communication with the air outlet and the atomization cavity respectively. A first capillary liquid absorbing structure is defined on the part of the inner wall of the atomization cavity connected to the inner wall of the air outlet channel, to absorb the aerosol substrate flowing back along the inner wall of the air outlet channel.

In some embodiments of the present disclosure, the first capillary liquid absorbing structure includes a first capillary groove and a second capillary groove. The first capillary groove is defined on the part of the inner wall of the atomization cavity connected to the inner wall of the air outlet channel. The second capillary groove is far from the air outlet channel relative to the first capillary groove and a gap exists between the second capillary groove and the first capillary groove. The aerosol substrate in the first capillary groove enters the second capillary groove after converging in the gap.

In some embodiments of the present disclosure, the width of each of the first capillary groove and the second capillary groove are less than 1 mm.

In some embodiments of the present disclosure, the first capillary groove and the second capillary groove are extended in a direction toward the air outlet channel.

In some embodiments of the present disclosure, the first capillary groove and the second capillary groove are extended in a direction toward the air outlet.

In some embodiments of the present disclosure, a second capillary liquid absorbing structure being extended to the atomization cavity is defined on the inner wall of the air outlet channel, to guide the aerosol substrate condensing on the inner wall of the air outlet channel to the atomization cavity.

In some embodiments of the present disclosure, a part of the second capillary liquid absorbing structure is in communication with the first capillary liquid absorbing structure defined on the part of the inner wall of the atomization cavity connected to the inner wall of the air outlet channel. The remaining part of the second capillary liquid absorbing structure and the first capillary liquid absorbing structure defined on the part of the inner wall of the atomization cavity connected to the inner wall of the air outlet channel are arranged at intervals.

In some embodiments of the present disclosure, a tapered channel is defined in the part of the atomization cavity in communication with the air outlet channel. The cross-sectional area of the tapered channel gradually decreases in a direction close to the air outlet channel.

In some embodiments of the present disclosure, the first capillary liquid absorbing structure defined on the part of the inner wall of the atomization cavity connected to the inner wall of the air outlet channel is at least partially located on the inner wall of the tapered channel.

In some embodiments of the present disclosure, the atomization assembly includes a porous heating body.

In some embodiments of the present disclosure, the porous heating body is a porous ceramic heating body.

The beneficial effect of the present disclosure is: compared with the related art, the present disclosure provides an atomization device. The first capillary liquid absorbing structure is defined on the part of the inner wall of the atomization cavity of the atomization device close to the atomization assembly. The first capillary liquid absorbing structure is configured to absorb the aerosol substrate, and after the first capillary liquid absorbing structure absorbs the aerosol substrate, the temperature of the cavity wall of the atomization cavity at the position where the first capillary liquid absorbing structure is located may be lowered with the endothermic atomization of the aerosol substrate, thereby avoiding the stability problems such as deformation and scorching of the cavity wall of the atomization cavity due to excessively high temperature, so that the structural stability of the atomization device may be improved.

Moreover, the first capillary liquid absorbing structure in the present disclosure has functions of absorbing and storing the aerosol substrate. The aerosol substrate on the cavity wall of the atomization cavity is at least partially locked in the first capillary liquid absorbing structure, thereby reducing the aerosol substrates accumulated in the atomization device, and further reducing the risk of liquid leakage of the atomization device, which is conducive to improving the anti-leakage effect of the atomization device.

### BRIEF DESCRIPTION OF THE DRAWINGS

Accompanying drawings herein are incorporated into a specification and constitute a part of this specification, show embodiments that conform to the present disclosure, and are used for describing a principle of the present disclosure together with this specification. In addition, the accompanying drawings and literal descriptions are not intended to limit the scope of the idea of the present disclosure in any manner, but explain the concept of the present disclosure by referring to specific embodiments for a person skilled in the art.
FIG. 1 is a schematic structural diagram of some embodiments of an atomization device in the present disclosure.
FIG. 2 is a cross-sectional view of the atomization device shown in FIG. 1 in an A-A direction.
FIG. 3 is a schematic structural diagram of some embodiments of an atomization assembly, a first carrier, and a second carrier of an atomization device in the present disclosure.
FIG. 4 is a schematic structural exploded view of the atomization assembly, the first carrier, and the second carrier shown in FIG. 3.
FIG. 5 is a schematic structural top view of the atomization assembly, the first carrier, and the second carrier shown in FIG. 3.
FIG. 6 is a schematic structural diagram of the atomization device shown in FIG. 2 after the atomization assembly is omitted.

### DETAILED DESCRIPTION

To make the objectives, technical solutions, and advantages of the present disclosure clearer, the following clearly and completely describes the technical solutions in the embodiments of the present disclosure with reference to the embodiments of the present disclosure. Apparently, the described embodiments are some rather than all of the embodiments of the present disclosure. All other embodiments obtained by a person of ordinary skill in the art based on the embodiments of the present disclosure without creative efforts shall fall within the protection scope of the present disclosure.

To resolve the technical problem of the poor structural stability and anti-leakage effect of the atomization device in the related art, some embodiments of the present disclosure provides an atomization device. The atomization device includes an air inlet, an air outlet, and an atomization cavity. The atomization cavity is in communication with the air inlet and the air outlet, and an atomization assembly is arranged in the atomization cavity. A first capillary liquid absorbing structure is defined on the part of the inner wall of the atomization cavity close to the atomization assembly. After the first capillary liquid absorbing structure absorbs an aerosol substrate, the temperature of the cavity wall of the atomization cavity at the position where the first capillary liquid absorbing structure is located may be lowered with endothermic atomization of the aerosol substrate. Detailed descriptions are provided below.

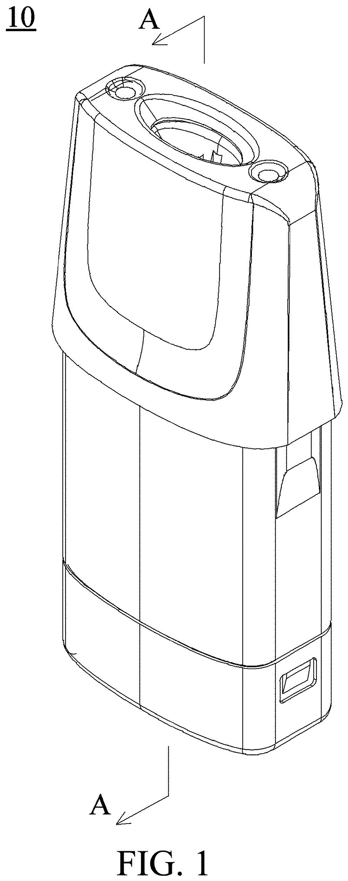

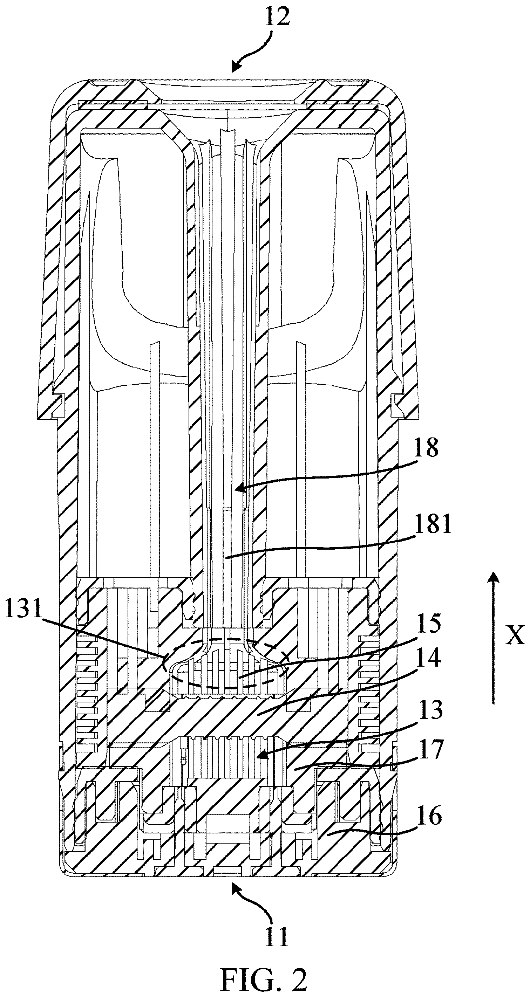

Referring to FIG. 1 and FIG. 2, FIG. 1 is a schematic structural diagram of some embodiments of an atomization device in the present disclosure, and FIG. 2 is a cross-sectional view of the atomization device shown in FIG. 1 in an A-A direction.

In some embodiments, the atomization device 10 may be in a form such as an e-cigarette. Certainly, the atomization device may also be a medical atomization device applied to the medical field. The atomization device 10 in the form of an e-cigarette is used as an example for description below. The present disclosure is not limited thereto.

Specifically, the atomization device 10 includes an air inlet 11, an air outlet 12, and an atomization cavity 13. The atomization cavity 13 is in communication with the air inlet 11 and the air outlet 12 respectively, and an atomization assembly 14 is arranged in the atomization cavity 13. The atomization assembly 14 is configured to atomize an aerosol substrate (such as e-liquid, liquid medicine, and so on) in the atomization device 10.

A position where the air inlet 11 is located is a position where air enters the atomization device 10. When a user inhales, external air enters the atomization cavity 13 from the air inlet 11, so that the aerosol substrate atomized by the atomization assembly 14 in the atomization cavity 13 is carried to the air outlet 12 and outputted to the user for the user to inhale.

In some embodiments, the atomization assembly 14 may be a porous heating body, which absorbs the aerosol substrate by a capillary force and generates heat to atomize the aerosol substrate. In some embodiments, the atomization assembly 14 may be a porous ceramic heating body or the like, and the atomization assembly 14 may also be arranged with a heating film. Certainly, in some embodiments of the present disclosure, the atomization assembly 14 may also be a design in which fiber cotton and heating wires are matched, which is not limited herein.

Considering that stability problems such as deformation and even scorching of the cavity wall of the atomization cavity 13 are usually caused when the temperature in the atomization cavity 13 is excessively high, in this embodiment, a first capillary liquid absorbing structure 15 is defined on the part of the inner wall of the atomization cavity 13 close to the atomization assembly 14. The first capillary liquid absorbing structure 15 is configured to absorb the aerosol substrate splashing to the cavity wall of the atomization cavity 13 due to oil frying, the aerosol substrate condensing on the cavity wall of the atomization cavity 13, and the like.

The atomization of the aerosol substrate requires heat absorption and the temperature of the position where the first capillary liquid absorbing structure 15 is located is high. Therefore, the aerosol substrate absorbed by the first capillary liquid absorbing structure 15 may continue to be atomized in the first capillary liquid absorbing structure 15. In this way, after the first capillary liquid absorbing structure 15 absorbs the aerosol substrate, the aerosol substrate in the first capillary liquid absorbing structure 15 continues to be atomized and the heat is at least partially absorbed by the aerosol substrate. Therefore, the heat of the cavity wall of the atomization cavity 13 transferred by the atomization assembly 14 to the position where the first capillary liquid absorbing structure 15 is located is reduced, and the heat of the cavity wall of the atomization cavity 13 at the position where the first capillary liquid absorbing structure 15 is located is absorbed by the aerosol substrate. Then, the temperature of the cavity wall of the atomization cavity 13 at the position where the first capillary liquid absorbing structure 15 is located is lowered. That is, the temperature of the cavity wall of the atomization cavity 13 at the position where the first capillary liquid absorbing structure 15 is located is lowered with the endothermic atomization of the aerosol substrate, which may avoid the stability problems such as deformation and scorching of the cavity wall of the atomization cavity 13 due to excessively high temperature, thereby improving the structural stability of the atomization device 10.

Certainly, that the aerosol substrate in the first capillary liquid absorbing structure 15 continues to be atomized also means the aerosol substrate absorbed by the first capillary liquid absorbing structure 15 is reused, which may improve the utilization of the aerosol substrate of the atomization device 10.

In some embodiments, when the atomization assembly 14 generates heat, the temperature of the cavity wall of the atomization cavity 13 is less than or equal to 150°C. It can be seen that, through the design of the first capillary liquid absorbing structure 15 in this embodiment, the temperature of the cavity wall of the atomization cavity 13 may be controlled to be below 150°C, thereby effectively avoiding the stability problems such as deformation and scorching of the cavity wall of the atomization cavity 13 due to excessively high temperature.

Further, the first capillary liquid absorbing structure 15 in this embodiment is defined on the part of the inner wall of the atomization cavity 13 close to the atomization assembly 14. That is, the first capillary liquid absorbing structure 15 is defined close to the atomization assembly 14, which may further reduce the risk of the stability problems of the cavity wall of the atomization cavity 13, thereby further improving the structural stability of the atomization device 10.

Moreover, the first capillary liquid absorbing structure 15 in this embodiment has functions of absorbing and storing the aerosol substrate. The aerosol substrate on the cavity wall of the atomization cavity 13 is at least partially locked in the first capillary liquid absorbing structure 15, thereby reducing the aerosol substrates accumulated in the atomization device 10, and further reducing the risk of liquid leakage of the atomization device 10, which is conducive to improving the anti-leakage effect of the atomization device 10. For example, for a direct-liquid atomization device 10, more aerosol substrates are usually accumulated at the bottom of the atomization device 10. Therefore, the first capillary liquid absorbing structure 15 in this embodiment may effectively reduce the aerosol substrates accumulated at the bottom of the atomization device 10, thereby reducing the risk of liquid leakage of the atomization device 10, and improving the anti-leakage effect of the atomization device 10.

It should be noted that when the user just starts to inhale, the aerosol substrate in the atomization assembly 14 has not been uniformly distributed, and the atomization assembly 14 has not been uniformly heated, that is, a local overheating case exits. Therefore, an oil frying phenomenon is most likely to occur at this time. Since the temperature of the atomization assembly 14 is low at this time, the temperature of the aerosol substrate splashing due to the oil frying is also low. Due to the low temperature, that the splashing aerosol substrate is absorbed by the first capillary liquid absorbing structure 15 does not affect the structural stability of the cavity wall of the atomization cavity 13 at the position where the first capillary liquid absorbing structure 15 is located. Instead, a protective film may be formed at the first capillary liquid absorbing structure 15 to reduce the temperature of the cavity wall of the atomization cavity 13 at the position where the first capillary liquid absorbing structure 15 is located with the endothermic atomization of the aerosol substrate in the first capillary liquid absorbing structure 15, thereby avoiding the stability problems such as deformation and scorching of the cavity wall of the atomization cavity 13 due to excessively high temperature.

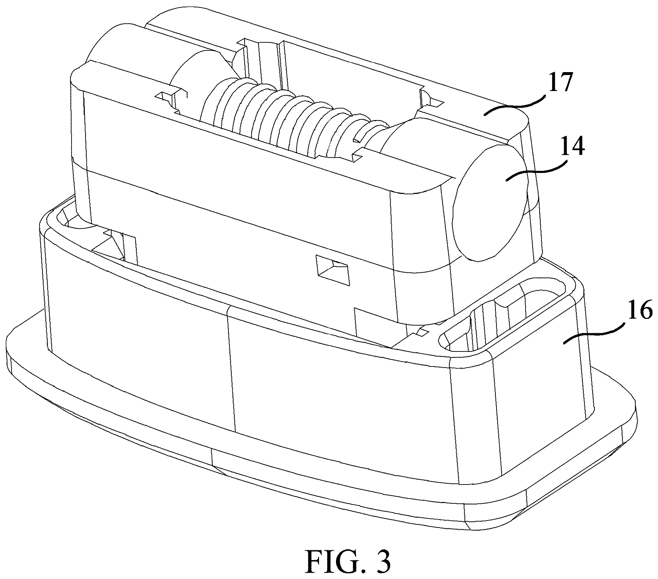

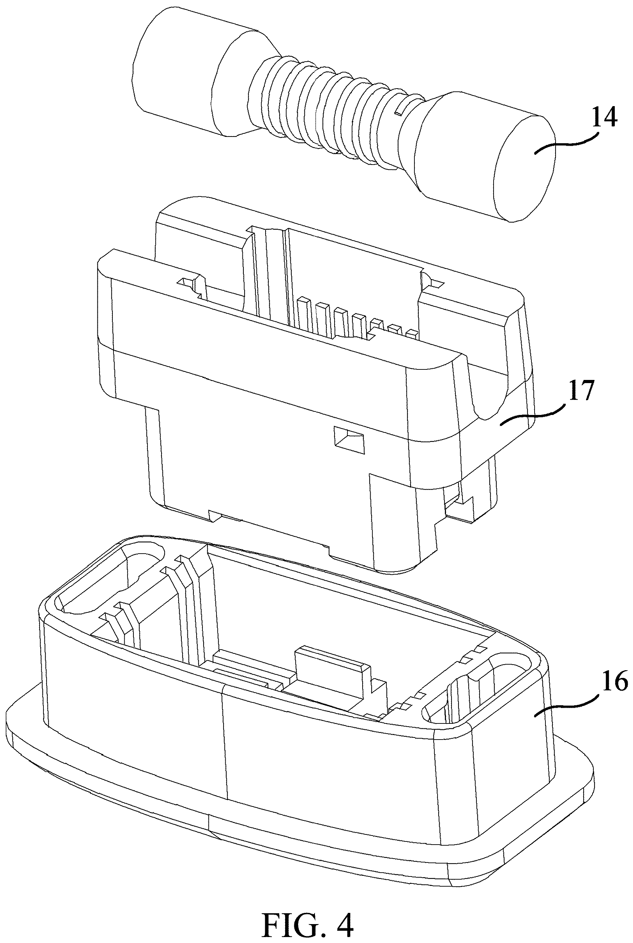

Referring to FIG. 2 to FIG. 4, FIG. 3 is a schematic structural diagram of some embodiments of an atomization assembly, a first carrier, and a second carrier of an atomization device in the present disclosure, and FIG. 4 is a schematic structural exploded view of the atomization assembly, the first carrier, and the second carrier shown in FIG. 3.

In some embodiments, the atomization device 10 further includes a first carrier 16 and a second carrier 17. The atomization cavity 13 is formed by docking the first carrier 16 and the second carrier 17. The air inlet 11 is provided on the first carrier 16. The atomization assembly 14 is arranged on the second carrier 17, and the first capillary liquid absorbing structure 15 is defined on the inner wall of the second carrier 17. The first capillary liquid absorbing structure 15 may be defined on the side wall or a bottom wall of the second carrier 17. FIG. 2 shows a case that the first capillary liquid absorbing structure 15 is defined on the side wall of the second carrier 17, which is not limited herein.

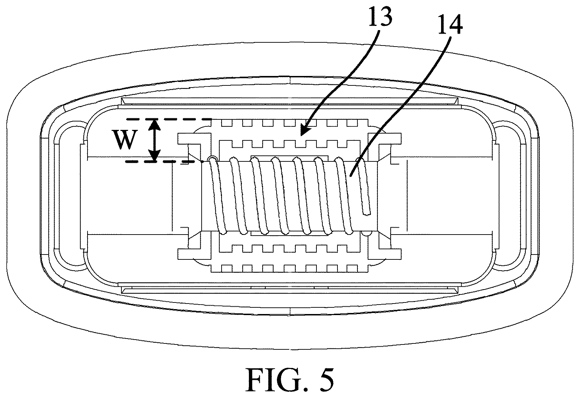

Referring to FIG. 2 and FIG. 5, FIG. 5 is a schematic structural top view of the atomization assembly, the first carrier, and the second carrier shown in FIG. 3.

In some embodiments, when the atomization assembly 14 heats up to its maximum temperature of 250°C, if the first capillary liquid absorbing structure 15 is not defined on the cavity wall of the atomization cavity 13, the distance between the surface of the atomization assembly 14 and the inner wall of the atomization cavity 13 usually needs to be more than 2 mm to ensure that the cavity wall of the atomization cavity 13 is not thermally deformed, scorched, or the like. This makes an excessively large cross-sectional area of the atomization cavity 13, and causes an excessively low flow velocity of an airflow in the atomization cavity 13 when the user inhales, which is not conducive for the airflow to carry the atomized aerosol substrate for the user to inhale.

In view of this, after the first capillary liquid absorbing structure 15 is defined on the cavity wall of the atomization cavity 13 in some embodiments, the distance (the distance W shown in FIG. 5) between the surface of the atomization assembly 14 and the inner wall of the atomization cavity 13 is allowed to decrease to a range of 0.5 mm to 1.8 mm, and then the temperature of the cavity wall of the atomization cavity 13 is controlled to be below 150°C. Compared with a case that the first capillary liquid absorbing structure 15 is not defined, the distance between the surface of the atomization assembly 14 and the inner wall of the atomization cavity 13 in this embodiment decreases, making the cross-sectional area of the atomization cavity 13 reduced while when the user inhales, and the flow velocity of the airflow in the atomization cavity 13 is increased. Specifically, when the user inhales, the flow velocity of the airflow in the atomization cavity 13 is at least increased by 10%, and it may be ensured that the cavity wall of the atomization cavity 13 is not thermally deformed, scorched, or the like. In addition, that the distance between the surface of the atomization assembly 14 and the inner wall of the atomization cavity 13 in this embodiment decreases means that the atomization cavity 13 is allowed to be designed with a smaller volume, which is conducive to the miniaturization of the atomization device 10.

In a case that the first capillary liquid absorbing structure 15 is defined on the cavity wall of the atomization cavity 13 in this embodiment, the distance between the surface of the atomization assembly 14 and the inner wall of the atomization cavity 13 ranges from 1 mm to 1.5 mm. In this way, the flow velocity of the airflow in the atomization cavity 13 may be increased as much as possible, and it may be ensured as much as possible that the cavity wall of the atomization cavity 13 is not thermally deformed, scorched, or the like.

Still referring to FIG. 2, in some embodiments, the atomization device 10 further includes an air outlet channel 18. The air outlet channel 18 is in communication with the air outlet 12 and the atomization cavity 13 respectively. Air entering the atomization cavity 13 from the air inlet 11 is carried to the air outlet channel 18 by the aerosol substrate atomized by the atomization assembly 14 and output to the user along the air outlet channel 18 for the user to inhale.

Condensation may occur after the atomized aerosol substrate contacts the inner wall of the air outlet channel 18, and the aerosol substrate condensing on the inner wall of the air outlet channel 18 may return to the atomization cavity 13 along the air outlet channel 18. Therefore, the first capillary liquid absorbing structure 15 is defined on the part of the inner wall of the atomization cavity 13 connected to the inner wall of the air outlet channel 18, to absorb the aerosol substrate flowing back along the inner wall of the air outlet channel 18. In this way, the aerosol substrate condensing on the inner wall of the air outlet channel 18 may be reused and configured to form the protective film at the first capillary liquid absorbing structure 15 to control the temperature of the cavity wall of the atomization cavity 13 (that is, reducing the temperature of the cavity wall of the atomization cavity 13 at the position where the first capillary liquid absorbing structure 15 is located with the endothermic atomization of the aerosol substrate in the first capillary liquid absorbing structure 15 as illustrated in the foregoing embodiment).

Further, to guide the aerosol substrate condensing on the inner wall of the air outlet channel 18 to flow back to the atomization cavity 13, a second capillary liquid absorbing structure 181 being extended to the atomization cavity 13 is defined on the inner wall of the air outlet channel 18 in this embodiment. After the second capillary liquid absorbing structure 181 absorbs the aerosol substrate condensing on the inner wall of the air outlet channel 18, the absorbed aerosol substrate is guided to the atomization cavity 13, which is conducive to optimizing a diversion effect of the aerosol substrate on the inner wall of the air outlet channel 18.

Further, a part of the second capillary liquid absorbing structure 181 is in communication with the first capillary liquid absorbing structure 15 defined on the part of the inner wall of the atomization cavity 13 connected to the inner wall of the air outlet channel 18. The aerosol substrate in the part of the second capillary liquid absorbing structure 181 is guided to the first capillary liquid absorbing structure 15 to help the accumulation of the aerosol substrates in the first capillary liquid absorbing structure 15, thereby improving the structural stability of the atomization device 10.

The remaining part of the second capillary liquid absorbing structure 181 and the first capillary liquid absorbing structure 15 defined on the part of the inner wall of the atomization cavity 13 connected to the inner wall of the air outlet channel 18 are arranged at intervals. That is, the remaining part of the second capillary liquid absorbing structure 181 is not in communication with the first capillary liquid absorbing structure 15. The aerosol substrate in the part of the second capillary liquid absorbing structure 181 may be directly guided to the atomization assembly 14 to be re-atomized by the atomization assembly 14.

Still referring to FIG. 2, in some embodiments, a tapered channel 131 is defined in the part of the atomization cavity 13 in communication with the air outlet channel 18. The cross-sectional area of the tapered channel 131 gradually decreases in a direction (as shown by the arrow X in FIG. 2, the same below) close to the air outlet channel 18. The first capillary liquid absorbing structure 15 defined on the part of the inner wall of the atomization cavity 13 connected to the inner wall of the air outlet channel 18 is at least partially located on the inner wall of the tapered channel 131.

When an obvious oil frying phenomenon occurs, the aerosol substrate of large droplets splashing vertically in the direction close to the air outlet channel 18 returns to the atomization assembly 14 under the action of gravity and is heated and re-atomized. A part of the aerosol substrate of large droplets splashing around is absorbed by the first capillary liquid absorbing structure 15 on the side. There is also a part of the aerosol substrate splashing upward obliquely in the direction close to the air outlet channel 18 at a vertical upward velocity, which may be blocked by the inner wall of the tapered channel 131 and absorbed by the first capillary liquid absorbing structure 15 on the inner wall of the tapered channel 131. The aerosol substrate of small droplets driven by the airflow may enter the air outlet channel 18 without being affected by the tapered channel 131. In this way, the aerosol substrate of large droplets may be prevented from entering the air outlet channel 18 to be inhaled by the user, avoiding affecting the user's taste.

Moreover, when the user inhales, the airflow passes through the tapered channel 131. The cross-sectional area of the tapered channel 131 gradually decreases in the direction close to the air outlet channel 18. Therefore, the flow velocity of the airflow in the tapered channel 131 is increased, which helps the atomized aerosol substrate to be carried and output to the user by the airflow, and further alleviates the condensation of the atomized aerosol substrate.

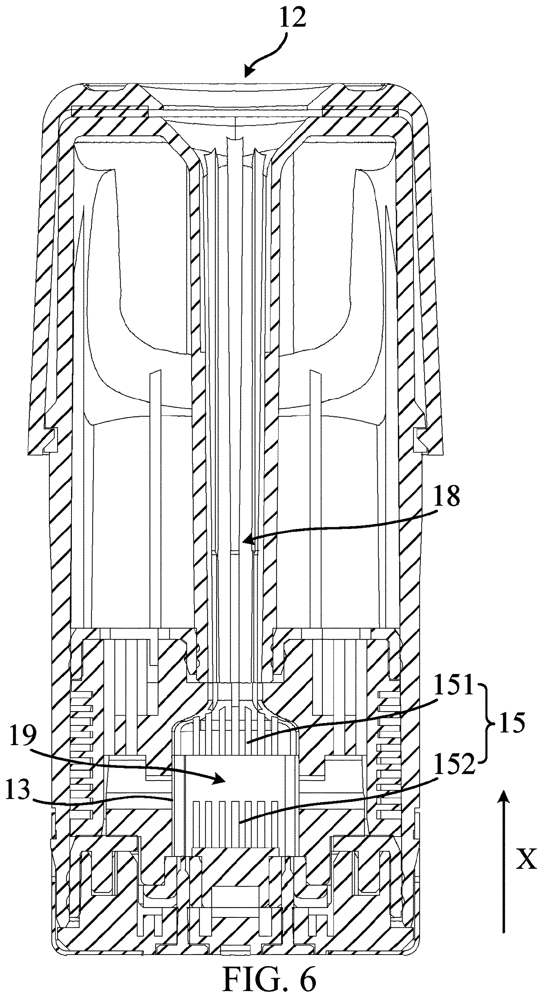

Referring to FIG. 6, FIG. 6 is a schematic structural diagram of the atomization device shown in FIG. 2 after the atomization assembly is omitted.

It should be noted that, the first capillary liquid absorbing structure 15 may be a capillary groove having a capillary force or the like, which may absorb the aerosol substrate by the capillary force. Certainly, the first capillary liquid absorbing structure 15 may also be other structures having the capillary force. For example, roughening processing such as grinding is performed on a surface of the atomization cavity 13, to form a structure having the capillary force in the form such as a frosted surface and a texture, that is, the first capillary liquid absorbing structure 15. The first capillary liquid absorbing structure 15 being the capillary groove is used as an example for description below. The type of the first capillary liquid absorbing structure 15 is not limited thereto.

In some embodiments, the first capillary liquid absorbing structure 15 includes a first capillary groove 151 and a second capillary groove 152. The first capillary groove 151 is provided on the part of the inner wall of the atomization cavity 13 connected to the inner wall of the air outlet channel 18. The second capillary groove 152 is far from the air outlet channel 18 relative to the first capillary groove 151. A gap 19 exits between the first capillary groove 151 and the second capillary groove 152. The aerosol substrate in the first capillary groove 151 enters the second capillary groove 152 after converging in the gap 19. In this way, the aerosol substrate in the first capillary groove 151 is allowed to be mixed uniformly at the gap 19 and be uniformly distributed to the second capillary groove 152, thereby improving the uniformity of the distribution of the aerosol substrate in the first capillary liquid absorbing structure 15 on the inner wall of the atomization cavity 13, which is conducive to reducing the risk of the structural stability problems of the cavity wall of the atomization cavity 13.

Further, the width of each of the first capillary groove 151 and the second capillary groove 152 are less than 1 mm, so that the first capillary groove 151 and the second capillary groove 152 have a sufficient capillary liquid absorbing capability. If the width of each of the first capillary groove 151 and the second capillary groove 152 are excessively large, the liquid absorbing capability of the first capillary groove 151 and the second capillary groove 152 may be weakened, which is insufficient for use. Moreover, a design value of the width of each of the first capillary groove 151 and the second capillary groove 152 further depends on the viscosity of the aerosol substrate and a structure design constraint of the atomization device 10. In addition, a larger depth of the capillary groove indicates a larger liquid storage capacity. Therefore, if the structure allows, increasing the depth of the capillary groove is conducive to increasing the liquid storage capacity of the first capillary groove 151 and the second capillary groove 152, thereby reducing the risk of liquid leakage.

Moreover, both the first capillary groove 151 and the second capillary groove 152 may be extended in the direction close to the air outlet channel 18, that is, both the first capillary groove 151 and the second capillary groove 152 are extended in a direction close to the air outlet 12, as shown by the arrow X in FIG. 6. Further, the first capillary groove 151 and the second capillary groove 152 are vertical grooves, which is conducive to injection molding of the first capillary groove 151 and the second capillary groove 152. Certainly, in other embodiments of the present disclosure, the first capillary groove 151 and the second capillary groove 152 may also not be vertical grooves, for example, may be implemented by a 3D printing technology.

To sum up, in the atomization device provided by the present disclosure, the first capillary liquid absorbing structure is defined on the part of the inner wall of the atomization cavity close to the atomization assembly. The first capillary liquid absorbing structure is configured to absorb the aerosol substrate, and after the first capillary liquid absorbing structure absorbs the aerosol substrate, the temperature of the cavity wall of the atomization cavity at the position where the first capillary liquid absorbing structure is located may be lowered with the endothermic atomization of the aerosol substrate, thereby avoiding the stability problems such as deformation and scorching of the cavity wall of the atomization cavity due to excessively high temperature, so that the structural stability of the atomization device may be improved.

Moreover, the first capillary liquid absorbing structure in the present disclosure has functions of absorbing and storing the aerosol substrate. The aerosol substrate on the cavity wall of the atomization cavity is at least partially locked in the first capillary liquid absorbing structure, thereby reducing the aerosol substrates accumulated in the atomization device, and further reducing the risk of liquid leakage of the atomization device, which is conducive to improving the anti-leakage effect of the atomization device.

In addition, in the present disclosure, unless otherwise explicitly specified or defined, the terms such as "connect", "connection" and "stack" should be understood in a broad sense. For example, the connection may be a fixed connection, a detachable connection, or an integral connection; or the connection may be a direct connection, an indirect connection through an intermediary, or internal communication between two components or mutual interaction relationship between two components. A person of ordinary skill in the art may understand the specific meanings of the foregoing terms in the present disclosure according to specific situations.

Finally, it should be noted that the foregoing embodiments are merely intended for describing the technical solutions of the present disclosure other than limiting the present disclosure. Although the present disclosure is described in detail with reference to the foregoing embodiments, a person of ordinary skill in the art should understand that they may still make modifications to the technical solutions described in the foregoing embodiments or make equivalent replacements to some or all technical features thereof, without departing defined by the appended claims. from the scope

## Claims

1. An atomization device (10), comprising:
an air inlet (11);
an air outlet (12); and
an atomization cavity (13), wherein the atomization cavity (13) is in communication with the air inlet (11) and the air outlet (12) respectively, and an atomization assembly (14) is arranged in the atomization cavity (13); a first capillary liquid absorbing structure (15) is defined on the part of the inner wall of the atomization cavity (13) close to the atomization assembly (14); and
**characterized in that** the atomization device is configured such that in response to the first capillary liquid absorbing structure (15) absorbing an aerosol substrate, the temperature of the cavity wall of the atomization cavity (13) at the position where the first capillary liquid absorbing structure (15) is located is lowered with endothermic atomization of the aerosol substrate.

2. The atomization device (10) according to claim 1, wherein the atomization device (10) further comprises a first carrier (16) and a second carrier (17); the atomization cavity (13) is formed by docking the first carrier (16) and the second carrier (17); the air inlet (11) is provided on the first carrier (16); the atomization assembly (14) is arranged on the second carrier (17); and the first capillary liquid absorbing structure (15) is defined on the inner wall of the second carrier (17).

3. The atomization device (10) according to claim 2, wherein the first capillary liquid absorbing structure (15) is defined on the side wall of the second carrier (17).

4. The atomization device (10) according to claim 1, wherein when the atomization assembly (14) generates heat, the temperature of the cavity wall of the atomization cavity (13) is less than or equal to 150°C.

5. The atomization device (10) according to claim 1, wherein the distance between the surface of the atomization assembly (14) and the inner wall of the atomization cavity (13) ranges from 0.5 mm to 1.8 mm.

6. The atomization device (10) according to claim 5, wherein the distance between the surface of the atomization assembly (14) and the inner wall of the atomization cavity (13) ranges from 1 mm to 1.5 mm.

7. The atomization device (10) according to claim 1, wherein the atomization device (10) further comprises an air outlet channel (18); the air outlet channel (18) is in communication with the air outlet (12) and the atomization cavity (13) respectively; and the first capillary liquid absorbing structure (15) is defined on the part of the inner wall of the atomization cavity (13) connected to the inner wall of the air outlet channel (18), to absorb the aerosol substrate flowing back along the inner wall of the air outlet channel (18).

8. The atomization device (10) according to claim 7, wherein the first capillary liquid absorbing structure (15) comprises a first capillary groove (151) and a second capillary groove (152); the first capillary groove (151) is provided on the part of the inner wall of the atomization cavity (13) connected to the inner wall of the air outlet channel (18); the second capillary groove (152) is far from the air outlet channel (18) relative to the first capillary groove (151) and a gap exists between the second capillary groove (152) and the first capillary groove (151); and the aerosol substrate in the first capillary groove (151) enters the second capillary groove (152) after converging in the gap.

9. The atomization device (10) according to claim 8, wherein the width of each of the first capillary groove (151) and the second capillary groove (152) are less than 1 mm.

10. The atomization device (10) according to claim 8, wherein the first capillary groove (151) and the second capillary groove (152) are extended in a direction toward the air outlet channel (18) or the air outlet (12).

11. The atomization device (10) according to claim 7, wherein a second capillary liquid absorbing structure (181) being extended to the atomization cavity (13) is defined on the inner wall of the air outlet channel (18).

12. The atomization device (10) according to claim 11, wherein a part of the second capillary liquid absorbing structure (181) is in communication with the first capillary liquid absorbing structure (15) defined on the part of the inner wall of the atomization cavity (13) connected to the inner wall of the air outlet channel (18), and the remaining part of the second capillary liquid absorbing structure (181) and the part of the first capillary liquid absorbing structure (15) defined on the part of the inner wall of the atomization cavity (13) connected to the inner wall of the air outlet channel (18) are arranged at intervals.

13. The atomization device (10) according to claim 7, wherein a tapered channel (131) is defined in the part of the atomization cavity (13) in communication with the air outlet channel (18), and the cross-sectional area of the tapered channel (131) gradually decreases in a direction toward the air outlet channel (18).

14. The atomization device (10) according to claim 13, wherein the first capillary liquid absorbing structure (15) defined on the part of the inner wall of the atomization cavity (13) connected to the inner wall of the air outlet channel (18) is at least partially located on the inner wall of the tapered channel (131).

15. The atomization device (10) according to claim 1, wherein the atomization assembly (14) comprises a porous heating body.

## Patentansprüche

1. Zerstäubungsvorrichtung (10), umfassend:
einen Lufteinlass (11);
einen Luftauslass (12); und
eine Zerstäubungskammer (13),
wobei die Zerstäubungskammer (13) jeweils mit dem Lufteinlass (11) und dem Luftauslass (12) in Verbindung steht und eine Zerstäubungsanordnung (14) in der Zerstäubungskammer (13) angeordnet ist und eine erste kapillare Flüssigkeitsabsorptionsstruktur (15) an dem Teil der Innenwand der Zerstäubungskammer (13) definiert ist, der sich in der Nähe der Zerstäubungsanordnung (14) befindet; und
**dadurch gekennzeichnet, dass** die Zerstäubungsvorrichtung derart konfiguriert ist, dass, wenn die erste kapillare Flüssigkeitsabsorptionsstruktur (15) ein Aerosolsubstrat absorbiert, die Temperatur der Kammerwand der Zerstäubungskammer an der Stelle, an der sich die erste kapillare Flüssigkeitsabsorptionsstruktur (15) befindet, durch die endotherme Zerstäubung des Aerosolsubstrats abgesenkt wird.

2. Die Zerstäubungsvorrichtung (10) gemäß Anspruch 1, wobei die Zerstäubungsvorrichtung (10) weiter einen ersten Träger (16) und einen zweiten Träger (17) umfasst; wobei die Zerstäubungskammer (13) durch das Andocken des ersten Trägers (16) und des zweiten Trägers (17) gebildet ist; wobei der Lufteinlass (11) an dem ersten Träger (16) vorgesehen ist; wobei die Zerstäubungsanordnung (14) an dem zweiten Träger (17) angeordnet ist; und wobei die erste kapillare Flüssigkeitsabsorptionsstruktur (15) an der Innenwand des zweiten Trägers (17) definiert ist.

3. Die Zerstäubungsvorrichtung (10) gemäß Anspruch 2, wobei die erste kapillare Flüssigkeitsabsorptionsstruktur (15) an der Seitenwand des zweiten Trägers (17) definiert ist.

4. Die Zerstäubungsvorrichtung (10) gemäß Anspruch 1, wobei, wenn die Zerstäubungsanordnung (14) Wärme erzeugt, die Temperatur der Kammerwand der Zerstäubungskammer (13) kleiner oder gleich 150 °C ist.

5. Die Zerstäubungsvorrichtung (10) gemäß Anspruch 1, wobei der Abstand zwischen der Oberfläche der Zerstäubungsanordnung (14) und der Innenwand der Zerstäubungskammer (13) zwischen 0,5 mm und 1,8 mm liegt.

6. Die Zerstäubungsvorrichtung (10) gemäß Anspruch 5, wobei der Abstand zwischen der Oberfläche der Zerstäubungsanordnung (14) und der Innenwand der Zerstäubungskammer (13) zwischen 1 mm und 1,5 mm liegt.

7. Die Zerstäubungsvorrichtung (10) gemäß Anspruch 1, wobei die Zerstäubungsvorrichtung (10) weiter einen Luftauslasskanal (18) umfasst, wobei der Luftauslasskanal (18) jeweils mit dem Luftauslass (12) und der Zerstäubungskammer (13) in Verbindung steht, und wobei die erste kapillare Flüssigkeitsabsorptionsstruktur (15) an dem Teil der Innenwand der Zerstäubungskammer (13) definiert ist, der mit der Innenwand des Luftauslasskanals (18) verbunden ist, um das Aerosolsubstrat zu absorbieren, das entlang der Innenwand des Luftauslasskanals (18) zurückfließt.

8. Die Zerstäubungsvorrichtung (10) gemäß Anspruch 7, wobei die erste kapillare Flüssigkeitsabsorptionsstruktur (15) eine erste Kapillarrille (151) und eine zweite Kapillarrille (152) umfasst; wobei die erste Kapillarrille (151) an dem Teil der Innenwand der Zerstäubungskammer (13) vorgesehen ist, der mit der Innenwand des Luftauslasskanals (18) verbunden ist; wobei die zweite Kapillarrille (152) im Vergleich zur ersten Kapillarrille (151) in einer vom Luftauslasskanal (18) weiter entfernten Position angeordnet ist, wobei ein Spalt zwischen der zweiten Kapillarrille (152) und der ersten Kapillarrille (151) ausgebildet ist; und wobei das in der ersten Kapillarrille (151) enthaltene Aerosolsubstrat in dem Spalt zusammenfließt, bevor es in die zweite Kapillarrille (152) eintritt.

9. Die Zerstäubungsvorrichtung (10) gemäß Anspruch 8, wobei die Breite sowohl der ersten Kapillarrille (151) als auch der zweiten Kapillarrille (152) kleiner als 1 mm ist.

10. Die Zerstäubungsvorrichtung (10) gemäß Anspruch 8, wobei sich die erste Kapillarrille (151) und die zweite Kapillarrille (152) in eine Richtung zum Luftauslasskanal (18) oder zum Luftauslass (12) erstrecken.

11. Die Zerstäubungsvorrichtung (10) gemäß Anspruch 7, wobei eine zweite kapillare Flüssigkeitsabsorptionsstruktur (181), die sich bis zur Zerstäubungskammer (13) erstreckt, an der Innenwand des Luftauslasskanals (18) definiert ist.

12. Die Zerstäubungsvorrichtung (10) gemäß Anspruch 11, wobei ein Teil der zweiten kapillaren Flüssigkeitsabsorptionsstruktur (181) mit der ersten kapillaren Flüssigkeitsabsorptionsstruktur (15) in Verbindung steht, die an dem Teil der Innenwand der Zerstäubungskammer (13) definiert ist, der mit der Innenwand des Luftauslasskanals (18) verbunden ist, und wobei der verbleibende Teil der zweiten kapillaren Flüssigkeitsabsorptionsstruktur (181) und der Teil der ersten kapillaren Flüssigkeitsabsorptionsstruktur (15), der an dem Teil der Innenwand der Zerstäubungskammer (13) definiert ist, der mit der Innenwand des Luftauslasskanals (18) verbunden ist, in Abständen angeordnet sind.

13. Die Zerstäubungsvorrichtung (10) gemäß Anspruch 7, wobei in dem mit dem Luftauslasskanal (18) in Verbindung stehenden Teil der Zerstäubungskammer (13) ein konischer Kanal (131) definiert ist und wobei sich die Querschnittsfläche des konischen Kanals (131) in Richtung zum Luftauslasskanal (18) allmählich verringert.

14. Die Zerstäubungsvorrichtung (10) gemäß Anspruch 13, wobei die erste kapillare Flüssigkeitsabsorptionsstruktur (15), die an dem Teil der Innenwand der Zerstäubungskammer (13) definiert ist, der mit der Innenwand des Luftauslasskanals (18) verbunden ist, sich mindestens teilweise an der Innenwand des konischen Kanals (131) befindet.

15. Die Zerstäubungsvorrichtung (10) gemäß Anspruch 1, wobei die Zerstäubungsanordnung (14) einen porösen Heizkörper umfasst.

## Revendications

1. Dispositif d'atomisation (10), comprenant :
une entrée d'air (11) ;
une sortie d'air (12) ; et
une cavité d'atomisation (13), la cavité d'atomisation (13) étant en communication respectivement avec l'entrée d'air (11) et la sortie d'air (12), un ensemble d'atomisation (14) étant disposé dans la cavité d'atomisation (13) et une première structure capillaire d'absorption de liquide (15) étant définie sur la partie de la paroi interne de la cavité d'atomisation (13) proche de l'ensemble d'atomisation (14) ; et
**caractérisé en ce que** le dispositif d'atomisation est configuré de sorte qu'en réponse à l'absorption d'un substrat d'aérosol par la première structure capillaire d'absorption de liquide (15), la température de la paroi de la cavité d'atomisation, à la position où se trouve la première structure capillaire d'absorption de liquide (15), est abaissée lors de l'atomisation endothermique du substrat d'aérosol.

2. Le dispositif d'atomisation (10) selon la revendication 1, dans lequel le dispositif d'atomisation (10) comprend en outre un premier support (16) et un second support (17) ; la cavité d'atomisation (13) étant formée par l'assemblage du premier support (16) et du second support (17) ; l'entrée d'air (11) étant disposée sur le premier support (16) ; l'ensemble d'atomisation (14) étant disposé sur le second support (17) et la première structure capillaire d'absorption de liquide (15) étant définie sur la paroi interne du second support (17).

3. Le dispositif d'atomisation (10) selon la revendication 2, dans lequel la première structure capillaire d'absorption de liquide (15) est définie sur la paroi latérale du second support (17).

4. Le dispositif d'atomisation (10) selon la revendication 1, dans lequel, lorsque l'ensemble d'atomisation (14) génère de la chaleur, la température de la paroi de la cavité d'atomisation (13) est inférieure ou égale à 150 °C.

5. Le dispositif d'atomisation (10) selon la revendication 1, dans lequel la distance entre la surface de l'ensemble d'atomisation (14) et la paroi interne de la cavité d'atomisation (13) est comprise entre 0,5 mm et 1,8 mm.

6. Le dispositif d'atomisation (10) selon la revendication 5, dans lequel la distance entre la surface de l'ensemble d'atomisation (14) et la paroi interne de la cavité d'atomisation (13) est comprise entre 1 mm et 1,5 mm.

7. Le dispositif d'atomisation (10) selon la revendication 1, le dispositif d'atomisation (10) comprenant en outre un canal de sortie d'air (18), le canal de sortie d'air (18) étant en communication respectivement avec la sortie d'air (12) et la cavité d'atomisation (13), et la première structure capillaire d'absorption de liquide (15) étant définie sur la partie de la paroi interne de la cavité d'atomisation (13) reliée à la paroi interne du canal de sortie d'air (18), afin d'absorber le substrat d'aérosol s'écoulant en retour le long de la paroi interne du canal de sortie d'air (18).

8. Le dispositif d'atomisation (10) selon la revendication 7, dans lequel la première structure capillaire d'absorption de liquide (15) comprend une première rainure capillaire (151) et une deuxième rainure capillaire (152) ; la première rainure capillaire (151) est disposée sur la partie de la paroi interne de la chambre d'atomisation (13) reliée à la paroi interne du canal de sortie d'air (18) ; la deuxième rainure capillaire (152) est, par rapport à la première rainure capillaire (151), située à une position plus éloignée du canal de sortie d'air (18), un espace étant formé entre la deuxième rainure capillaire (152) et la première rainure capillaire (151) ; et le substrat d'aérosol contenu dans la première rainure capillaire (151) conflue dans l'espace avant de pénétrer dans la deuxième rainure capillaire (152).

9. Le dispositif d'atomisation (10) selon la revendication 8, dans lequel la largeur de chacune de la première rainure capillaire (151) et de la deuxième rainure capillaire (152) est inférieure à 1 mm.

10. Le dispositif d'atomisation (10) selon la revendication 8, dans lequel la première rainure capillaire (151) et la deuxième rainure capillaire (152) s'étendent dans une direction allant vers le canal de sortie d'air (18) ou vers la sortie d'air (12).

11. Le dispositif d'atomisation (10) selon la revendication 7, dans lequel une deuxième structure capillaire d'absorption de liquide (181), s'étendant jusqu'à la chambre d'atomisation (13), est définie sur la paroi interne du canal de sortie d'air (18).

12. Le dispositif d'atomisation (10) selon la revendication 11, dans lequel une partie de la deuxième structure capillaire d'absorption de liquide (181) est en communication avec la première structure capillaire d'absorption de liquide (15) définie sur la partie de la paroi interne de la chambre d'atomisation (13) reliée à la paroi interne du canal de sortie d'air (18), et dans lequel la partie restante de la deuxième structure capillaire d'absorption de liquide (181) et la partie de la première structure capillaire d'absorption de liquide (15) définie sur la partie de la paroi interne de la chambre d'atomisation (13) reliée à la paroi interne du canal de sortie d'air (18) sont disposées à intervalles.

13. Le dispositif d'atomisation (10) selon la revendication 7, dans lequel un canal conique (131) est défini dans la partie de la chambre d'atomisation (13) qui est en communication avec le canal de sortie d'air (18), et dans lequel la surface de section transversale du canal conique (131) diminue progressivement dans une direction allant vers le canal de sortie d'air (18).

14. Le dispositif d'atomisation (10) selon la revendication 13, dans lequel la première structure capillaire d'absorption de liquide (15), définie sur la partie de la paroi interne de la chambre d'atomisation (13) reliée à la paroi interne du canal de sortie d'air (18), est au moins partiellement située sur la paroi interne du canal conique (131).

15. Le dispositif d'atomisation (10) selon la revendication 1, dans lequel l'ensemble d'atomisation (14) comprend un corps chauffant poreux.
